# EUROPÄISCHE PATENTANMELDUNG

(11) **EP 1 084 707 A1**
(43) Veröffentlichungstag der Anmeldung: **21.03.2001**
(21) Anmeldenummer: 00119902.5
(22) Anmeldetag: 13.09.2000
(51) Int. Cl.: A61K 35/74, C12N 1/20, C12R 1/19

(54) **Probiotischer E. Coli-Stamm**

(30) Priorität: 13.09.1999 DE 19943644
(71) Anmelder: Feinchemie GmbH Sebnitz, D-01855 Sebnitz (DE)
(72) Erfinder: Reissbrodt, Rolf, Dr. rer. nat. habil., 38855 Wernigerode (DE)
(74) Vertreter: Draudt, Jutta, Dr.

(57) **Zusammenfassung**

Es werden ein probiotischer *E.coli* 015:H⁻-Stamm und eine pharmazeutische Zusammensetzung, die einen probiotischen *E.coli-*Stamm enthält, beschrieben.

## Beschreibung

Die vorliegende Erfindung betrifft einen *E.coli* 015:H⁻-Stamm als probiotisches Mittel und eine probiotische pharmazeutische Zusammensetzung.

Pathogene *Escherichia coli* (EHEC)-Bakterien verursachen beim Menschen schwere Durchfallerkrankungen mit wässrigen und blutigen Stühlen; als postinfektiöse Erkrankungen können sie das hämolytisch-urämische Syndrom (HUS) und thrombotischthrombocytopenische Purpura (TTP) verursachen. EHEC-Stämme wirken im Wesentlichen toxisch durch ihre Fähigkeit, Shigatoxine zu produzieren. Die Shigatoxinproduktion wird *in vitro* und *in vivo* durch Antibiotikagabe erhöht. Deshalb ist eine Antibiotikabehandlung bei EHEC-Infektionen kontraindiziert. Die Therapie besteht gegenwärtig im Ausgleich der Flüssigkeits- und Elektolytdefiziens und bei den postinfektiösen Erkrankungen werden die Patienten meistens an die Dialyse angeschlossen.

Die intestinale Mikroflora hat eine essentielle Bedeutung auf den Gesundheitszustand des Menschen. Eine positive Beeinflussung dieser Mikroflora bei gesundheitlichen Störungen, insbesondere des Verdauungstraktes, durch Zugabe von lebenden und abgetöteten apathogenen Mikroorganismen ist seit längerer Zeit bekannt. Sie wird in Form der sogenannten Probiotika bei Mensch und Tier angewendet. Die therapeutische Wirkung der dadurch eintretenden Modifizierung der Mikroflora des Darmes sind eine Immunmobilisation und eine Erhöhung der Mukosa-Abwehr gegen pathogene Mikroorganismen.

Es bestand daher ein Bedarf dahingehend, ein Mittel zur Verfügung zu stellen, das eine spezielle Wirkung auf die Shigatoxinproduktion und auf das Wachstum von enterohämorrhagischen *E.coli* im Darmtrakt aufweist.

Diese Aufgabe wird durch den *E.coli* 015:H⁻-Stamm nach Anspruch 1 gelöst.

Es hat sich erfindungsgemäß erwiesen, dass der *E.coli* 015:H⁻-Stamm bzw. das *E.coli* 015:H⁻-Bakterium als probiotisches Mittel hervorragend geeignet ist, die Shigatoxinproduktion bzw. das Wachstum von enterohämorrhagischen *E.coli*-Stämmen (EHEC) zu hemmen. In einer bevorzugten Ausführungsform der vorliegenden Erfindung hemmt der *E.coli* 015:H⁻-Stamm die Shigatoxine von Shigellen.

Die erfindungsgemäße pharmazeutische Zusammensetzung enthält, neben üblichen Zusatzstoffen, einen probiotischen *E.coli-*Stamm. In einer bevorzugten Ausführungsform ist der probiotische *E.coli*-Stamm der erfindungsgemäßen pharmazeutischen Zusammensetzung der *E.coli* 015:H⁻-Stamm.

Die pharmazeutische Zusammensetzung kann als Verdünnungsmittel einen Zucker enthalten. Als Zucker lässt sich bspw. Lactose oder Glucose verwenden. Es kann aber auch jeder andere Zucker enthalten sein, der für die Konfektionierung der vorliegenden pharmazeutischen Zusammensetzung geeignet ist. Die Konzentrationen der einzelnen Bestandteile liegen in den üblichen Grenzen und sind dem Fachmann auf dem hier betreffenden Gebiet geläufig.

Es hat sich herausgestellt, dass der probiotiosche *E.coli-*Stamm in der pharmazeutischen Zusammensetzung bevorzugt in lyophilisierter Form vorliegt. Die Lyophilisierung wird in üblicher Weise aus dem flüssigen Produkt durchgeführt.

Vor der Verarbeitung wird das Präparat in üblicher Weise aufgelöst und auf eine geeignete Verdünnung gebracht.

Des Weiteren hat sich herausgestellt, dass ohne Weiteres in der erfindungsgemäßen pharmazeutischen Zusammensetzung andere probiotische Präparate, die im Handel erhältlich sind, zugemischt sein können.

Der aus einer Stuhlprobe isolierte und mikrobiologisch, biochemisch und genetisch charakterisierte probiotische *E.coli* ist in der Lage, in Bouillon-Mischkulturen und in Stuhlproben mit enterohämorrhagischen *E.coli* (EHEC) die Shigatoxinproduktion bzw. das Wachstum der EHEC-Stämme zu hemmen (Tab. 1 - 3, Abb. 1). Die Hemmung wurde bei Mischungen des probiotischen *E.coli* zum EHEC-Stamm schon in einem Verhältnis von 1:10 gemessen. Dazu wurde die Shigatoxinproduktion in den Mischkulturen durch Shigatoxin-ELISA bestimmt. Die Hemmung (%) bezieht sich immer auf die Shigatoxinproduktion der EHEC-Stämme ohne Zusatz des probiotischen *E.coli*.

Die Hemmung wurde bei Mischungen des probiotischen *E.coli* zum EHEC-Stamm schon in einem Verhältnis von 1:10 gemessen, sie betraf alle geprüften EHEC-Serovare (*E.coli* O157 und *E.coli* non-O157). Neben der Hemmung der Shigatoxinproduktion kommt es auch zu einer Reduzierung der Zellzahl der EHEC-Stämme, wenn sie mit dem probiotischen *E.coli* in Mischkultur angezüchtet wurden.

Die Wirkung wird bei frischen Kulturen des probiotischen *E.coli* und bei Anwendung eines lyophilisierten Trockenpräparates erreicht. Besonders in der letzteren Form ist es als Präparat anzuwenden.

Ein bevorzugter probiotischer *E.coli*-Stamm ist der *E.coli* O15:H⁻-Stamm. Dieser Stamm trägt keine der für pathogene *E.coli* bekannten Gene (für Enterotoxin, Shigatoxin, keine *eaeA, ipaH, astA, α-hly, ehx, pap, sfa-fimC*) und ist in der Lage, die Shigatoxinproduktion enterohämorrhagischer *E.coli* (EHEC) oder deren Wachstum in Bouillonkulturen und im Stuhl zu hemmen. Der probiotische *E.coli* bildet H₂S, spaltet aber in H₂S-positive und H₂S-negative Kolonien, die beide gleich wirksam sind. Die probiotische Wirkung auf die Shigatoxinproduktion oder das Wachstum der EHEC-Stämme wird mit frischen Bakterienkulturen und nach Lyophilisierung des probiotischen *E.coli*-Stammes erreicht.

Das nachfolgende Beispiel dient der Erläuterung der Erfindung.

### Beispiel:

Die Untersuchungen zur Hemmung der Shigatoxinproduktion von EHEC-Stämmen wurden in Bouillonkulturen oder im Stuhl als Schüttelkulturen (Kulturmedium EHEC Toxinmedium direkt (Heipha)) bei 37°C, 18 bis 20 Std. ausgeführt. Die in den Tabellen 1 und 3 und der Abbildung 1 angeführten EHEC-Stämme wurden mit dem probiotischen *E.coli* als Mischkultur angezüchtet. Alle Stämme wurden vorher frisch kultiviert, die Zellzahlen mit der MPN-Methode bestimmt. In den Kulturüberständen wurden die produzierten Shigatoxine mit Shigatoxin-ELISA Kits des Handels (z. B. Ridascreen® Verotoxin-ELISA, r-biopharm, Darmstadt) geprüft. In prinzipiell gleicher Weise wurde mit dem lyophilisierten Trockenpräparat (nach Zählung der Überlebensraten nach der Lyophilisierung) des probiotischen *E.coli* verfahren (Tab. 2).

Die Ergebnisse sind in den Tabellen 1 bis 3 und in der Abb. 1 dargestellt.

Die Reduktion des Wachstums der EHEC-Stämme wurde in Mischkultur mit dem probiotischen *E.coli* durch Bestimmung der Zellzahlen an enterohämolytischen EHEC-Kolonien im Verhältnis zur Gesamtzellzahl in Bouillonkulturen nach Ausplattieren auf Enterohämolysinagar Heipha ermittelt. Die Zellzahlen reduzierten sich auf ca. 1/10 bis 1/100 der ohne probiotischen *E.coli* bestimmten Zellzahlen.

**Tab. 1**

| **Hemmung der Shigatoxinproduktion von EHEC-Stämmen angezüchtet in Bouillonkultur mit dem probiotischen** ***E. coli*** | | | | |
|---|---|---|---|---|
| **Stamm-Nr.** | **Serovar** | **Shigatoxine** | | **Hemmung** |
| | | **1** | **2** | |
| EDL 933 | O157:H7 | + | + | 63 % |
| CDC 933 | O157:H7 | + | - | 89 % |
| 97-10085 | O157:H7 | - | + | 98 % |
| 97-05622 | O157:H7 | + | + | 46 % |
| 97-05289 | O8,60:H5 | + | + | 91 % |
| 97-07922 | O145:H⁻ | - | + | 84 % |
| 97-10084 | O100:H⁻ | - | + | 85 % |
| 97-10581-1 | O137:H⁻ | + | - | > 99 % |
| 97-16157 | O103:H2 | + | - | 98 % |
| 97-07772 | O26:H⁻ | - | + | 68 % |
| 97-14606 | O30:H⁻ | + | + | > 99 % |

**Tab. 2**

| **Hemmung der Shigatoxinproduktion eines EHEC-Stammes durch Inkubation mit dem probiotischen *E.coli* als lyophilisierte Trockensubstanz in Bouillonkultur** | | | |
|---|---|---|---|
| **Zellzahlen des EHEC-Stammes 97-14606** | **Trockenmasse des probiotischen *E.coli*** | **Zellzahlen des probiotischen** ***E.coli*** | **Hemmung** |
| 125 | 4 µg | ca. 5 · 10³ | 92 % |
| 125 | 4 ng | 15 | 58 % |

**Tab. 3**

| **Wirkung der kommensalen** ***E.coli*****-Stämme und eines** ***Lactobacillus acidophilus*****-Stammes auf die Hemmung der Shigatoxinproduktion** | | | |
|---|---|---|---|
| **Stamm** | **Anzahl der geprüften Stämme** | **Zellzahlen der geprüften Stämme und Zellzahlen das EHEC- Stammes 97-14606** | **Hemmung** |
| *E.coli*-Isolate von Stuhlproben | 3 | ca. 20:200 | 0 |
| *E.coli*-Stämme des Präparates Symbioflor® 2 | 6 | ca. 200:200 | ca. 10 % |
| *Lactobacillus acidophilus* | 1 | ca. 200:200 | 0 |

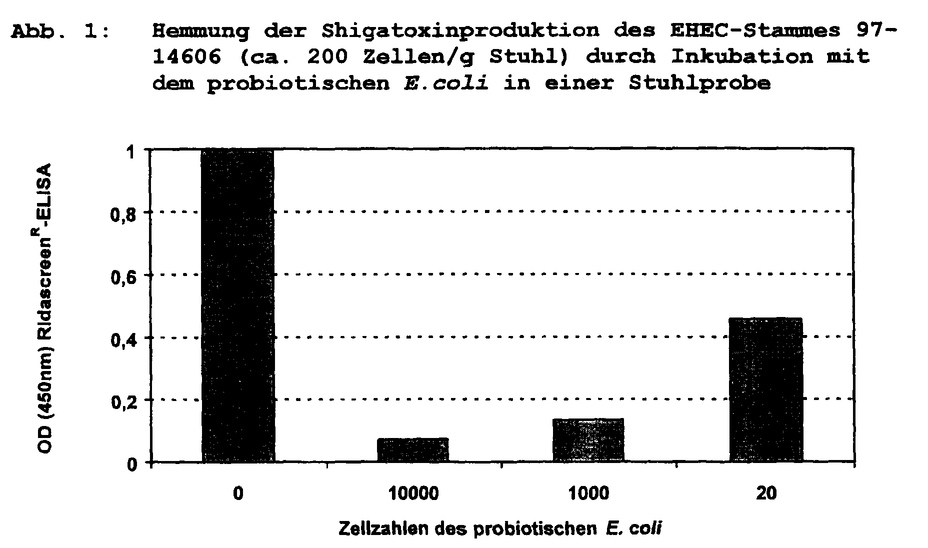

## Patentansprüche

1. *E.coli* 015:H⁻-Stamm als probiotisches Mittel zur Hemmung der Shigatoxinproduktion und des Wachstums von enterohämorrhagischen *E.coli*-Stämmen (EHEC).

2. E.coli-Stamm nach Anspruch 1, dadurch gekennzeichnet, dass er Shigatoxine von Shigellen hemmt.

3. Pharmazeutische Zusammensetzung, die einen probiotischen *E.coli*-Stamm enthält.

4. Pharmazeutische Zusammensetzung nach Anspruch 3, dadurch gekennzeichnet, dass der probiotische *E.coli*-Stamm der *E.coli* 015:H⁻-Stamm ist.

5. Pharmazeutische Zusammensetzung nach Anspruch 3 oder 4, dadurch gekennzeichnet, dass sie einen Zucker, vorzugsweise Lactose oder Glucose enthält.

6. Pharmazeutische Zusammensetzung nach mindestens einem der Ansprüche 3 bis 5, dadurch gekennzeichnet, dass der probiotische *E.coli*-Stamm in lyophilisierter Form vorliegt.

7. Pharmazeutische Zusammensetzung nach mindestens einem der Ansprüche 3 bis 6, dadurch gekennzeichnet, dass andere probiotische Präparate zugemischt sind.

8. Verwendung eines probiotischen *E.coli*-Stammes in flüssiger oder fester Form in einem probiotischen Präparat in der Human- oder Veterinärmedizin zur Hemmung der von enterohämorrhagischen *E.coli* (EHEC) produzierten Shigatoxine.

9. Verwendung nach Anspruch 8, dadurch gekennzeichnet, dass die Shigatoxine von Shigellen gehemmt werden.

10. Verwendung nach Anspruch 8, dadurch gekennzeichnet, dass das Wachstum von pathogenen Bakterien wie enterohämorrhagische *E.coli* und anderer pathogener Bakterien reduziert wird.

11. Verwendung nach mindestens einem der Ansprüche 8 bis 10, dadurch gekennzeichnet, dass der probiotische *E.coli*-Stamm der *E.coli* 015:H⁻-Stamm ist.
